# EUROPÄISCHE PATENTSCHRIFT

(11) **EP 2 525 718 B1**
(45) Veröffentlichungstag und Bekanntmachung des Hinweises auf die Patenterteilung: **25.03.2020**
(21) Anmeldenummer: 10794910.9
(22) Anmeldetag: 22.11.2010
(51) Int. Cl.: A61B 10/00, G01N 33/52, G01N 33/84

(54) **INDIKATIONSEINRICHTUNG**
INDICATOR DEVICE
DISPOSITIF D'INDICATION

(30) Priorität: 19.01.2010 DE 102010001032
(43) Veröffentlichungstag der Anmeldung: 28.11.2012
(73) Patentinhaber: Aristotech Holding GmbH, 10719 Berlin (DE)
(72) Erfinder: ANAPLIOTIS, Emmanuel, 14195 Berlin (DE)
(74) Vertreter: Gulde & Partner
(86) Internationale Anmeldenummer: PCT/EP2010/067916
(87) Internationale Veröffentlichungsnummer: WO 2011/088920

(56) Entgegenhaltungen:
- EP-A1- 1 346 692
- US-A- 3 954 563
- US-A- 5 147 288
- US-A- 5 869 003
- US-A1- 2008 009 769
- US-B1- 6 186 946
- US-B1- 6 409 680

## Beschreibung

Die vorliegende Erfindung betrifft eine Indikationseinrichtung zur Indikation zumindest eines chemischen Wertes einer Flüssigkeit, insbesondere einer Körperflüssigkeit, wobei die Indikationseinrichtung wenigstens ein zur Kontaktierung mit der Flüssigkeit vorgesehenes erstes Indikations-Element umfasst, welches auf einem Träger angeordnet ist, der eine Längserstreckung aufweist, die mindestens dem Dreifachen seiner maximalen Quererstreckung entspricht.

Die erfindungsgemäße Indikationseinrichtung ist insbesondere zur Indikation des pH-Wertes von Körperflüssigkeiten eingerichtet und lässt sich vorzugsweise aufgrund ihrer langgestreckten Form zur Bestimmung des Säuregehaltes des Scheidenmilieus einsetzen.

Eine der häufigsten Ursachen von vaginalen Infektionen und dadurch ausgelösten Frühgeburten ist die Besiedlung der Scheide mit Bakterien, die Infektionen bewirken. Derartige Veränderungen des Scheidenmilieus werden oftmals von den Schwangeren nicht bemerkt. Die fremden Bakterien verdrängen die natürlicherweise in der Scheide vorhandenen Milchsäure-Bakterien, welche durch Bildung von Milchsäuren ein saures Scheidenmilieu herstellen. Das saure Scheidenmilieu stellt einen natürlichen Schutz gegen das Eindringen fremder Bakterien dar. Eine Veränderung des natürlichen Gleichgewichtes zu Ungunsten des Milchsäuregehaltes führt zu einer messbaren Änderung des pH-Wertes. Das heißt, dass durch Bestimmung des pH-Wertes Rückschlüsse gezogen werden können auf fremde, schädliche Bakterien. Je früher eine pathologische Änderung des Scheidenmilieus aufgrund fremder, Infektionen auslösender Bakterien erkannt wird, umso eher können Gegenmaßnahmen durch den behandelnden Arzt zur Vermeidung von Frühgeburten eingeleitet werden.

Eine unnormale Erhöhung des Säuregehaltes des Scheidenmilieus kann dagegen auf eine Beschädigung der Fruchtblase hinweisen, bei der geringe Mengen von Fruchtwasser in einen oberen Scheidenbereich gelangen. Das ausgetretene Fruchtwasser führt zu einer Erhöhung des pH-Wertes. Das heißt, dass bei einer unnormalen Erhöhung des pH-Wertes Rückschlüsse gezogen werden können auf eine möglicherweise beschädigte Fruchtblase. Demzufolge können frühzeitig Behandlungsmaßnahmen vorgenommen werden.

Zur Feststellung des pH-Wertes ist ein Wattestäbchen bekannt, welches über seine gesamte Länge mit einer den Säuregehalt anzeigenden Flüssigkeit imprägniert oder getränkt ist. Aufgrund seiner schlanken Form lässt sich dieses Wattestäbchen vorteilhaft zur Bestimmung des pH-Wertes im oberen vaginalen Bereich verwenden. Durch Kontakt mit der oberen Schleimhaut der Vagina wechselt es seine Farbe.

Es besteht insbesondere bei Schwangeren neben der Feststellung des pH-Wertes des Scheidenmilieus außerdem die Anforderung bzw. das Bedürfnis zur Entnahme von Körperflüssigkeiten zwecks Anfertigung eines Abstriches. Dazu ist es gegebenenfalls notwendig, Flüssigkeiten aus der Scheide zu entnehmen, um sie einer allgemeinen mikrobiologischen Untersuchung zuzuführen. Da das genannte Wattestäbchen über seine gesamte Länge mit der Indikationsflüssigkeit getränkt bzw. imprägniert ist, ist es nicht geeignet, Körperflüssigkeiten für einen Abstrich aufzunehmen, da die Körperflüssigkeiten mit der Indikationsflüssigkeit vermischt werden könnten.

Zur Bestimmung des pH-Wertes von Körperflüssigkeiten ist außerdem ein Handschuh bekannt, der aus einem leichten, flüssigkeitsundurchlässigen Material besteht und an dessen einen Fingerkuppenbereich ein Indikationsstreifen angeordnet ist. Nachteilig an dieser Einrichtung ist, dass der Indikationsstreifen sehr weit vorn an einem Handschuhfinger angeordnet ist, so dass nur eine zu kleine oder gar keine Fläche zwischen Indikationsstreifen und Handschuhfingerspitze zur Aufnahme von Körperflüssigkeiten zur Verfügung steht. Außerdem ist ein mit Körperflüssigkeit benetzter Handschuh nur umständlich einer mikrobakteriellen Untersuchung in einem Labor zuführbar.

Eine weitere bekannte Einrichtung zur Bestimmung des pH-Wertes, mit der gegebenenfalls auch Körperflüssigkeit für einen Abstrich aufgenommen werden kann, ist ein längliches Trägerelement, welches mit einer Schutzhülle überzogen ist, auf der ein Indikationsstreifen angeordnet ist. Diese Einrichtung weist allerdings den Nachteil der relativ hohen Herstellungskosten sowie aufgrund gegebenenfalls bei Gebrauch erfolgender Faltung bzw. Knitterung der Schutzhülle die Vermittlung eines unangenehmen Gefühles auf.

Aus dem Dokument US 2008/0009769 A1 ist eine Einrichtung zur Bestimmung des pH-Wertes bekannt, welche mit einer Einrichtung zur Detektion von Aminen kombiniert ist. Ein Nachteil dieser Einrichtung ist, dass zur Anfertigung eines Abstriches eine extra Einrichtung eingesetzt werden müsste und ein gesonderter Diagnoseprozess durchgeführt werden müsste.

Die US 3 954 563 A beschreibt eine Vorrichtung zur Untersuchung von weiblichen Personen auf Gonokokken, die eine Entnahme eines Abstrichs und Mittel zur chemischen Auswertung in einer kompakten Einheit zusammenfasst.

Die EP 1 346 692 A1 beschreibt eine Vorrichtung zum Testen von Hygienezuständen, zum Beispiel in Restaurants, durch Entnahme eines Schwammabstrichs.

Die US 5 147 288 A offenbart eine Vorrichtung zur Reinigung eines Gehörganges.

Gegenstand der US 5 869 003 A ist eine diagnostische Testeinheit mit einer Probenkammer, in der eine Analyse stattfindet und die mit einer Verschlusskappe versehen ist.

Aus der US 2008/009769 A1 geht ein pH-Schnelltest für weibliche Personen hervor, dessen Struktur aus einem Griffstück und einem länglichen Teil besteht, an dem mehrere Teststreifen angebracht sein können. Es können also mehrere Tests mit Indikatoren gleichzeitig durchgeführt werden.

Die US 6 186 946 B1 beschreibt eine Vorrichtung zur Urinanalyse bei Tieren, weiche eine Form eines länglichen flachen Plättchens hat, an dessen einen Ende ein verfärbbarer Abschnitt vorgesehen ist.

Aus der US 6 409 680 B1 gehen ein Träger und ein Detektor zur Detektion eines pH-Werts und einem Vorhandensein von pathogenen Bakterien hervor. Ein Indikations-Element ist auf dem Träger angeordnet und ein Schwamm zur Entnahme eines Abstrichs ist an einem entgegengesetzten Ende des Trägers angeordnet.

Der vorliegenden Erfindung liegt somit die Aufgabe zugrunde, eine Einrichtung zur Verfügung zu stellen, mit der nach nur einer Kontaktierung mit einer Körperflüssigkeit gleichzeitig und somit zeit- und kostensparend mehrere Untersuchungen durchgeführt werden können.

Diese Aufgabe wird erfindungsgemäß durch die Indikationseinrichtung nach Anspruch 1 gelöst. Vorteilhafte Ausgestaltungen sind in den Unteransprüchen 2 bis 11 angegeben. Die Erfindung ergänzend wird eine Verwendung der Indikationseinrichtung gemäß Anspruch 12 zur Verfügung gestellt.

Erfindungsgemäß wird eine Indikationseinrichtung zur Indikation zumindest eines chemischen Wertes einer Flüssigkeit, insbesondere einer Körperflüssigkeit, zur Verfügung gestellt, welche wenigstens ein zur Kontaktierung mit der Flüssigkeit vorgesehenes erstes Indikations-Element umfasst, das auf einem Träger angeordnet ist, der eine Längserstreckung aufweist, die mindestens dem Dreifachen seiner maximalen Querersteckung entspricht. Erfindungsgemäß ist an einem ersten Ende des Trägers ein Abstrich-Element, welches bevorzugt ein Wattenstäbchen aus Baumwolle ist, zur zumindest kurzzeitigen Aufnahme der Flüssigkeit angeordnet.

E ist vorgesehen, dass der Träger zumindest abschnittsweise die Form eines Hohlzylinders aufweist und das Abstrich-Element einen Stift aufweist, welcher zumindest abschnittsweise in den Hohlzylinder gesteckt oder steckbar ist, so dass ein mit dem Stift verbundener Aufnahmebereich des Abstrich-Elementes zur Aufnahme der Flüssigkeit aus dem Hohlzylinder herausragt, wobei das Abstrich-Element am Stift als Aufnahmebereich einen ersten Wattebausch aufweist, dessen maximale Quererstreckung größer ist als der Innendurchmesser des Hohlzylinders.

Der chemische Wert ist dabei bevorzugt ein biochemischer Wert, wie zum Beispiel ein pH-Wert. Das heißt, dass die erfindungsgemäße Indikationseinrichtung vorzugsweise eine Vorrichtung zur Messung des pH-Wertes ist. Die Aufnahme der Flüssigkeit am Abstrich-Element kann durch Adhäsion erfolgen. Das Abstrich-Element kann dabei ein integraler Bestandteil des Trägers sein. Das erste Ende des Trägers, an dem das Abstrich-Element angeordnet ist, ist dabei ein Ende des Trägers in Richtung seiner Längserstreckung. Das Verhältnis von Längserstreckung zur Quererstreckung bedingt eine längserstreckte Form des Trägers und somit eine Stift- oder Zapfenform. Die äußere Form des Trägers ist nicht eingeschränkt auf einen runden Querschnitt, sondern der Träger kann gegebenenfalls auch einen eckigen Querschnitt, in diesem Fall vorzugsweise mit abgerundeten Kanten, aufweisen. In einer bevorzugten Ausführungsform ist das Indikations-Element direkt auf dem Träger angeordnet und kontaktiert den Träger somit unmittelbar.

Das Indikations-Element ist, wie bereits näher erläutert, vorzugsweise ein pH-Indikationsstreifen zur optischen Bestimmung des Säuregrades der Flüssigkeit. Das heißt, dass das Indikations-Element als Flächenelement und vorteilhafterweise als Klebeband ausgeführt ist. Durch die Anordnung des Indikations-Elementes auf dem Träger in der Nähe des Abstrich-Elementes am vorderen Ende des Trägers ist dieser in einfacher Weise in die Scheide einführbar, wobei die Bestimmung des Säuregehaltes des oberen Scheidenmilieus gleichzeitig mit der Entnahme einer geringen Menge Körperflüssigkeit erfolgen kann. Nach Herausziehen aus der Scheide lässt sich der Säuregehalt anhand einer möglicherweise stattgefundenen Farbänderung des Indikations-Elementes feststellen und die am Abstrich-Element anhaftende Körperflüssigkeit einer Untersuchung, insbesondere einer mikrobiologischen Untersuchung, zuführen. Die Schwangere selbst bzw. der behandelnde Arzt können die erfindungsgemäße Indikationseinrichtung zur Feststellung des Säuregehaltes der Vaginalflora und zur Durchführung eines Abstriches verwenden. Bei Feststellung eines unnormalen Säuregehaltes lässt sich die entnommene Körperflüssigkeit sogleich auf einen unnormalen Bakteriengehalt untersuchen und/oder es können Untersuchungen der Fruchtblase eingeleitet werden. Dadurch kann die Gefahr von Frühgeburten erheblich vermindert werden. Der Vorteil der Erfindung liegt insbesondere in der Gleichzeitigkeit der Bestimmung des Säuregehaltes und der Entnahme von Körperflüssigkeit.

Der Träger kann aus demselben Material hergestellt sein wie das Indikations-Element. Das heißt, dass auch ein vom Träger umfasster Griffbereich aus dem Indikationsmaterial hergestellt ist. Dies ist somit eine alternative Ausführungsform zu der Ausgestaltung der Erfindung, in der das Indikations-Element auf dem Träger befestigt ist.

Dadurch, dass der Träger zumindest abschnittsweise die Form eines Hohlzylinders aufweist und das Abstrich-Element einen Stift aufweist, welcher zumindest abschnittsweise in den Hohlzylinder gesteckt oder steckbar ist, so dass ein mit dem Stift verbundener Aufnahmebereich des Abstrich-Elementes zur Aufnahme der Flüssigkeit aus dem Hohlzylinder herausragt, ist in einfacher Weise die Kombination von einem Indikations-Element auf dem Träger und einem Abstrich-Element am Ende des Trägers durch Einstecken des Stiftes in den Hohlzylinder des Trägers realisierbar. Der hohlzylinderförmige Träger ist dabei vorzugsweise aus Pappe oder einem anderen biologisch abbaubaren Material hergestellt, wobei das Indikations-Element als Klebeband auf dem Träger aufgeklebt ist.

Durch die maximale Quererstreckung des Wattebausches, die größer ist als der Innendurchmesser des Hohlzylinders, ist einem in den Hohlzylinder eingesteckten Abstrich-Element ein translatorischer Freiheitsgrad in einer Richtung genommen, wodurch ein ungewolltes Einschieben des Wattbausches in den Hohlzylinder des Trägers verhindert wird.

In einer alternativen Ausführungsform ist vorgesehen, dass das Abstrich-Element durch den Träger ausgebildet ist. Das Abstrich-Element ist dann somit ein integraler Bestandteil des Trägers.

In einer alternativen Ausführungsform ist vorgesehen, dass das Abstrich-Element aus einem anderen Material als der Träger hergestellt ist. In dieser Ausführungsform ist das Abstrich-Element kein integraler Bestandteil des Trägers, sondern zum Beispiel ein an den Träger anschließender Wattebausch. Das Abstrich-Element ist somit auch nicht als Indikations-Element ausgebildet.

In einer weiteren bevorzugten Ausführungsform schließt das Indikations-Element direkt an das Abstrich-Element an.

Dabei können das Indikations-Element und das Abstrich-Element aus gleichen oder unterschiedlichen Materialien hergestellt sein. Das Indikations-Element ist somit ebenfalls am ersten Ende des Trägers angeordnet, jedoch ausgehend vom Abstrich-Element hinter diesem positioniert. Das heißt, dass das Abstrich-Element das Ende der Indikationseinrichtung ausbildet, wobei das Indikations-Element zum Abstrich-Eiement benachbart angeordnet ist.

Die Ausgestaltung mit Wattebausch kann dabei durch einen zweiten Wattebausch erweitert sein, so dass das Abstrich-Element beidseitig am Stift einen Wattebausch aufweist. Vorzugsweise ragen dabei beide Wattebäusche aus dem Träger heraus bzw. stehen von diesem ab, so dass die Indikationseinrichtung beidseitig für einen Abstrich verwendbar ist. Ein herkömmliches Wattestäbchen mit beidseitig angeordneten Wattebäuschen kann somit zur Herstellung der erfindungsgemäßen Indikationseinrichtung verwendet werden.

In einer besonderen Ausführungsform der Erfindung ist vorgesehen, dass das Indikations-Element ebenfalls mit einem Stift und daran anschließenden zweiten Wattebausch ausgestaltet ist, wobei der Stift des Indikations-Elementes ebenfalls in den Hohlzylinder gesteckt oder steckbar ist. Somit sind der erste und der zweite Wattebausch nebeneinander am ersten Ende des Trägers angeordnet. Der Wattebausch des Indikations-Elementes ist dabei vorzugsweise mit einem den pH-Wert einer Flüssigkeit anzeigenden Indikator getränkt.

Das zweite Ende des Trägers, welches dem ersten Ende gegenüberliegt, ist vorzugsweise als manuell nutzbarer Griff ausgestaltet. Das heißt, dass wenigstens das zweite Ende des Trägers derart stabil ausgestaltet ist, dass es manuell gegriffen und die gesamte Indikationseinrichtung in die Scheide eingeführt und herausgezogen werden kann.

Zwecks Einklemmung und/oder formschlüssiger Fixierung eines in einem hohlzylinderförmigen Träger aufgenommenen Stiftes ist vorgesehen, dass der Hohlzylinder an wenigstens einer Stelle eine Einengung seines hohlen Querschnittes in zumindest einer quer zur Hohlzylinderlängsachse verlaufenden Richtung aufweist. Diese Einengung soll dabei derart geformt sein, dass ein engstes Maß des eingeengten Querschnittes geringer ist als das maximale Maß der Quererstreckung eines Abstrich-Elementes, insbesondere für die Situation, in der das Abstrich-Element als Wattestäbchen in den als Träger dienenden Hohlzylinder eingeschoben ist. Damit erfolgt eine formschlüssige Arretierung des Abstrich-Elementes im Hohlzylinder in einem translatorischen Freiheitsgrad in Trägerlängsrichtung. Die Fixierung des Abstrich-Elementes kann dabei gegebenenfalls durch eine Klebung zwischen Träger und Abstrich-Element im gepressten bzw. eingedrückten Bereich verbessert werden.

Zur Vermeidung von Kanten bzw. Absätzen an der Indikationseinrichtung ist vorgesehen, dass der Hohlzylinder des Trägers und das Abstrich-Element im Wesentlichen den gleichen Durchmesser aufweisen. Bei Verwendung von im Wesentlichen eckig geformten Abstrich-Elementen und/oder Trägern sollte die maximale Quererstreckung vom Träger bzw. vom Abstrich-Element im Wesentlichen gleich sein. Damit wird die gleichzeitige Benetzung des Abstrich-Elementes und des Indikations-Elementes erleichtert und eine gegebenenfalls leichte Drehbewegung des Trägers nicht als unangenehm empfunden. Das Indikations-Element berührt dabei die Haut der Scheide und wird im Bereich des Abstrich-Elementes gleichzeitig befeuchtet.

In bevorzugten Ausführungsformen sind das Abstrich-Element und/ oder das Indikations-Element und/oder der Träger aus einem biologisch abbaubaren Material hergestellt.

Dabei müssen nicht alle drei genannten Elemente der Indikationseinrichtung aus demselben umweltfreundlichen Material hergestellt sein. Es können Naturmaterialien wie z. B. Baumwolle, Papier, Pappe oder andere aus Biopolymeren hergestellte Materialien eingesetzt werden.

Neben der erfindungsgemäßen Indikationseinrichtung wird erfindungsgemäß außerdem die Verwendung der Indikationseinrichtung zur Indikation zumindest eines chemischen Wertes einer Flüssigkeit und zur gleichzeitigen Flüssigkeitsentnahme zur Durchführung eines Abstriches zur Verfügung gestellt. Die erfindungsgemäße Indikationseinrichtung wird vorzugsweise zur Indikation eines pH-Wertes einer Flüssigkeit und zur Entnahme der Flüssigkeit, insbesondere Scheidenflüssigkeit, verwendet.

Die vorliegende Erfindung wird im Folgenden anhand der beiliegenden Zeichnungen erläutert. Es zeigen:
- Figur 1: eine erfindungsgemäße Indikationseinrichtung erster Ausführungsform in Ansicht von der Seite,
- Figur 2: einen Träger einer Indikationseinrichtung in Ansicht von der Seite,
- Figur 3: ein Abstrich-Element erster Ausführungsform in Ansicht von der Seite,
- Figur 4: ein Abstrich-Element zweiter Ausführungsform in Ansicht von der Seite,
- Figur 5: eine Indikationseinrichtung zweiter Ausführungsform in Ansicht von der Seite, und
- Figur 6: eine Indikationseinrichtung dritter Ausführungsform in Ansicht von der Seite.

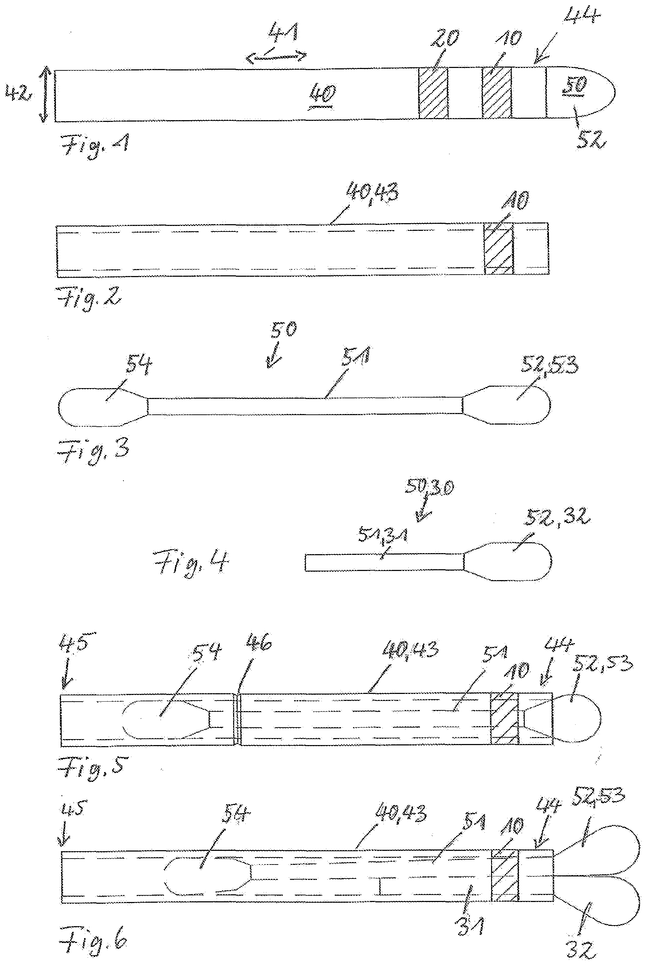

In Figur 1 ist eine Indikationseinrichtung dargestellt, bei der deutlich die Stift- bzw. Stabform erkennbar ist. Das heißt, es ist ersichtlich, dass die Längserstreckung 41 wesentlich größer ist als die Quererstreckung 42. Es ist dabei an einem länglichen Träger 40 an dessen ersten Ende 44 ein Abstrich-Element 50 angeordnet. Dieses Abstrich-Element 50 weist einen Aufnahmebereich 52 zur Aufnahme von Flüssigkeit auf. Auf dem Träger 40 ist in der Nähe des ersten Endes 44 ein erstes Indikations-Element 10 angebracht. Dieses erste Indikations-Element 10 kann zum Beispiel ein Indikatorstreifen, vorzugsweise selbstklebend, sein. Dabei ist die Erfindung nicht auf den Abstand zwischen dem ersten Indikations-Element 10 und dem Abstrich-Element 50 eingeschränkt, sondern es kann das erste Indikations-Element 10 auch direkt an das Abstrich-Element 50 anschließen. Es ist ersichtlich, dass die Indikationseinrichtung leicht in Körperöffnungen, wie zum Beispiel in eine Scheide, einführbar ist, in der sie mit Körperflüssigkeit benetzbar ist, so dass der pH-Wert der Flüssigkeit vom ersten Indikations-Element 10 angezeigt werden kann und am Aufnahmebereich 52 des Abstrich-Elementes 50 anhaftende Körperflüssigkeit einer Untersuchung zugeführt werden kann.

Wie in Figur 1 dargestellt kann außerdem beabstandet oder direkt an das erste Indikations-Element 10 angrenzend ein zweites Indikations-Element 20 angeordnet sein, mit welchem ebenfalls ein chemischer Wert der Körperflüssigkeit anzeigbar ist. Dieses zweite Indikations-Element 20 kann dabei ebenfalls ein pH-Indikator zum Vergleich mit dem ersten Indikations-Element 10 sein, oder ein Indikations-Element zur Anzeige eines anderen Wertes.

Der Aufbau der Indikationseinrichtung in unterschiedlichen Ausführungsformen ist aus den Figuren 2 bis 6 ersichtlich.

In Figur 2 ist ein Träger 40 dargestellt, der die Form eines Hohlzylinders 43 aufweist. Mit den gestrichelten Linien ist die Innenwandung des Hohlzylinders 43 angedeutet. Ein derartiger Träger ist vorzugsweise aus Pappe oder einem anderen, biologisch abbaubaren Material herstellbar.

Aus Figur 3 ist eine alternative Ausführungsform eines Abstrich-Elementes 50 erkennbar, welches einen Stift 51 aufweist, an dem beidseitig Wattebäusche angeordnet sind, nämlich an einem ersten Ende der erste Wattebausch 53 und am gegenüberliegenden Ende der zweite Wattebausch 54. Für ein derartiges Abstrich-Element 50 kann ein herkömmliches Wattestäbchen verwendet werden. Die Wattebäusche 53, 54 stellen somit auch die jeweiligen Aufnahmebereiche 52 dar. Das Abstrich-Element 50 kann derart in einem in Figur 2 dargestellten Träger 40 angeordnet sein, dass die beiden Wattebäusche 53, 54 beidseitig aus dem Träger 40 herausragen (nicht dargestellt). Bei größeren Durchmessern der Wattebäusche 53, 54 als der Innendurchmesser des Hohlzylinders 43 ist das Abstrich-Element 50 somit formschlüssig zumindest in seinen translatorischen Freiheitsgraden im Träger 40 fixiert.

Eine alternative Ausführungsform des Abstrich-Elementes 50 ist in Figur 4 dargestellt, wobei an einem kürzeren Stift 51 nur einseitig ein Aufnahmebereich 52, hier ebenfalls in Form eines Wattebausches, angeordnet ist. Ein solches Abstrich-Element 50 kann ebenfalls in dem hohlzylinderförmigen Träger 40 gemäß Figur 2 aufgenommen werden, wobei er zur Fixierung in diesem Träger 40 vorzugsweise zu kleben bzw. zu klemmen ist.

Die in Figur 4 dargestellte Einrichtung kann jedoch auch als drittes Indikations-Element 30 verwendet werden, welches einen Stiel 31 und einen daran angeschlossenen Wattebausch 32 ausweist. Dieses dritte Indikations-Element 30 kann, wie in Figur 6 dargestellt ist, neben einem Abstrich-Element 50 nach Figur 3 in einen hohlzylinderförmigen Träger 40 eingeschoben sein, so dass an einem ersten Ende 44 des Trägers 40 beide Wattebäusche 53, 32 angeordnet sind. Der erste Wattebausch 53 des Abstrich-Elementes 50 dient in beschriebener Weise zur Aufnahme von Körperflüssigkeit. Der Wattebausch des dritten Indikations-Elementes 32 ist mit einer Indikationsfiüssigkeit getränkt bzw. imprägniert und dient zur Anzeige eines chemischen Wertes, insbesondere eines pH-Wertes.

In Figur 5 ist eine besonders bevorzugte Ausführungsform der Erfindung dargestellt, bei der im Träger 40 eine Einengung 46 vorgesehen ist, die zu einem verringerten Innenquerschnitt des Hohlzylinders 43 führt, so dass die Wattebäusche 53, 54 des Abstrich-Elementes 50 nicht aus dem Träger 40 in Richtung der Einengung 46 gezogen werden können. Dabei ist die vorliegende Erfindung nicht darauf eingeschränkt, dass der zweite Wattebausch 54, wie in den Figuren 5 und 6 dargestellt, im Träger 40 angeordnet ist, sondern er kann auch am zweiten Ende des Trägers 45 aus diesem herausragen.

Die Einengung 46 kann dabei derart dimensioniert sein, dass es zu einer Berührung der Innenwand des Hohlzylinders 43 des Trägers 40 mit dem Stift 51 kommt und zumindest eine leichte Presspassung zwischen diesen Bauteilen realisiert ist. Somit ist zwischen einer formschlüssigen Fixierung und Positionierung des Abstrich-Elementes 50 im Träger 40 außerdem eine kraftschlüssige Fixierung realisiert. Zur weiteren Erhöhung der Festigkeit der mechanischen Verbindung zwischen Träger 40 und Abstrich-Element 50 kann zwischen diesen Bauteilen eine Klebung, vorzugsweise im Bereich der Einengung 46, vorgenommen sein.

### Bezugszeichenliste

- 10: erstes Indikations-Element

- 20: zweites Indikations-Element

- 30: drittes Indikations-Element
- 31: Stift des dritten Indikations-Elementes
- 32: Wattebausch des dritten Indikations-Elementes

- 40: Träger
- 41: Längserstreckung
- 42: Quererstreckung
- 43: Hohlzylinder

- 44: erstes Ende des Trägers
- 45: zweites Ende des Trägers
- 46: Einengung

- 50: Abstrich-Element
- 51: Stift
- 52: Aufnahmebereich
- 53: erster Wattebausch
- 54: zweiter Wattebausch

## Patentansprüche

1. Indikationseinrichtung zur Indikation zumindest eines chemischen Wertes einer Flüssigkeit, insbesondere einer Körperflüssigkeit, umfassend wenigstens ein zur Kontaktierung mit der Flüssigkeit vorgesehenes erstes Indikations-Element (10), welches auf einem Träger (40) angeordnet ist, der eine Längserstreckung (41) aufweist, die mindestens dem Dreifachen seiner maximalen Quererstreckung (42) entspricht, wobei an einem ersten Ende des Trägers (44) ein Abstrich-Element (50) zur zumindest kurzzeitigen Aufnahme der Flüssigkeit angeordnet ist, **dadurch gekennzeichnet, dass** der Träger (40) zumindest abschnittsweise die Form eines Hohlzylinders (43) aufweist und das Abstrich-Element (50) einen Stift (51) aufweist, welcher zumindest abschnittsweise in den Hohlzylinder (43) gesteckt oder steckbar ist, so dass ein mit dem Stift (51) verbundener Aufnahmebereich (52) des Abstrich-Elementes (50) zur Aufnahme der Flüssigkeit aus dem Hohlzylinder (43) herausragt,
und dass das Abstrich-Element (50) am Stift (51) als Aufnahmebereich (52) einen ersten Wattebausch (53) aufweist, dessen maximale Quererstreckung größer ist als der Innendurchmesser des Hohlzylinders (43).

2. Indikationseinrichtung nach Anspruch 1,
**dadurch gekennzeichnet,**
**dass** das Indikations-Element (10) ein pH-Indikationsstreifen ist zur optischen Bestimmung des Säuregrades der Flüssigkeit.

3. Indikationseinrichtung nach einem der Ansprüche 1 oder 2,
**dadurch gekennzeichnet,**
**dass** der Träger (40) aus demselben Material hergestellt ist wie das Indikations-Element (10).

4. Indikationseinrichtung nach wenigstens einem der Ansprüche 1 bis 3,
**dadurch gekennzeichnet,**
**dass** das Abstrich-Element (50) aus einem anderen Material als der Träger (40) hergestellt ist.

5. Indikationseinrichtung nach wenigstens einem der vorhergehenden Ansprüche,
**dadurch gekennzeichnet,**
**dass** das Indikations-Element (10) direkt an das Abstrich-Element (50) anschließt.

6. Indikationseinrichtung nach wenigstens einem der vorhergehenden Ansprüche,
**dadurch gekennzeichnet,**
**dass** das Abstrich-Element (50) beidseitig am Stift (51) einen Wattebausch (53, 54) aufweist.

7. Indikationseinrichtung nach wenigstens einem der vorhergehenden Ansprüche,
**dadurch gekennzeichnet,**
**dass** das Indikations-Element (10) ebenfalls mit einem Stift (51) und daran anschließenden zweiten Wattebausch (54) ausgestaltet ist, wobei der Stift (51) des Indikations-Elementes (10) ebenfalls in den Hohlzylinder (43) gesteckt oder steckbar ist.

8. Indikationseinrichtung nach wenigstens einem der vorhergehenden Ansprüche,
**dadurch gekennzeichnet,**
**dass** das zweite Ende des Trägers (40) als manuell nutzbarer Griff ausgestaltet ist.

9. Indikationseinrichtung nach wenigstens einem der vorhergehenden Ansprüche,
**dadurch gekennzeichnet,**
**dass** der Hohlzylinder (43) an wenigstens einer Stelle eine Einengung seines hohlen Querschnitts in zumindest einer senkrecht zur Hohlzylinder (43)-Längsrichtung verlaufenden Richtung aufweist.

10. Indikationseinrichtung nach wenigstens einem der vorhergehenden Ansprüche,
**dadurch gekennzeichnet,**
**dass** der Hohlzylinder (43) und das Abstrich-Element (50) im Wesentlichen den gleichen Durchmesser aufweisen.

11. Indikationseinrichtung nach wenigstens einem der vorhergehenden Ansprüche,
**dadurch gekennzeichnet,**
**dass** das Abstrich-Element (50) und/oder das Indikations-Element (10) und/oder der Träger (40) aus einem biologisch abbaubaren Material hergestellt ist.

12. Verwendung der Indikationseinrichtung nach wenigstens einem der Ansprüche 1 bis 11, zur Indikation zumindest eines chemischen Wertes einer Flüssigkeit und zur gleichzeitigen Flüssigkeitsentnahme zur Durchführung eines Abstriches.

## Claims

1. An indication device for indicating at least one chemical value of a liquid, in particular of a bodily fluid, comprising at least one first indication element (10) provided for contacting with the liquid, said indication element being arranged on a carrier (40) which has a longitudinal extension (41) corresponding to at least three times its maximum transverse extension (42), wherein a swab element (50) is arranged at a first end of the carrier (44) for at least short-term reception of the liquid,
**characterized in that**
the carrier (40) has, at least in portions, the shape of a hollow cylinder (43) and the swab element (50) has a pin (51) which, at least in portions, is inserted or insertable into the hollow cylinder (43), such that a reception area (52) of the swab element (50) that is connected to the pin (51) protrudes from the hollow cylinder (43) for reception of the liquid,
and
**in that** the swab element (50) has a first cotton ball (53) on the pin (51) as a reception area (52), said cotton ball having a maximum transverse extension which is larger than the interior diameter of the hollow cylinder (43).

2. The indication device according to Claim 1,
**characterized in that**
the indication element (10) is a pH indicator strip for optically determining the acidity of the liquid.

3. The indication device according to any one of Claims 1 or 2,
**characterized in that**
the carrier (40) is manufactured from the same material as the indication element (10).

4. The indication device according to at least one of Claims 1-3,
**characterized in that**
the swab element (50) is manufactured from a different material than the carrier (40).

5. The indication device according to at least one of the preceding claims,
**characterized in that**
the indication element (10) directly adjoins the swab element (50).

6. The indication device according to at least one of the preceding claims,
**characterized in that**
the swab element (50) has a cotton ball (53, 54) on both sides of the pin (51).

7. The indication device according to at least one of the preceding claims,
**characterized in that**
the indication element (10) is also configured with a pin (51) and a second cotton ball (54) attached thereto, the pin (51) of the indication element (10) also being inserted or insertable into the hollow cylinder (43).

8. The indication device according to at least one of the preceding claims,
**characterized in that**
the second end of the carrier (40) is configured as a manually usable handle.

9. The indication device according to at least one of the preceding claims,
**characterized in that**
the hollow cylinder (43) has a constriction of its hollow cross-section in at least one place in at least one direction running perpendicularly to the longitudinal direction of the hollow cylinder (43).

10. The indication device according to at least one of the preceding claims,
**characterized in that**
the hollow cylinder (43) and the swab element (50) have substantially the same diameter.

11. The indication device according to at least one of the preceding claims,
**characterized in that**
the swab element (50) and/or the indication element (10) and or the carrier (40) is/are manufactured from a biodegradable material.

12. Use of the indication device according to at least one of Claims 1 to 11 for indicating at least one chemical value of a liquid and for simultaneous liquid removal for carrying out swabbing.

## Revendications

1. Dispositif d'indication pour indiquer au moins une valeur chimique d'un liquide, notamment d'un liquide biologique, comprenant au moins un premier élément d'indication (10) prévu pour la mise en contact avec le liquide, lequel est disposé sur un support (40), qui présente une étendue longitudinale (41), qui correspond au moins au triple de son étendue transversale maximale (42), dans lequel un élément de frottis (50) est disposé à une première extrémité du support (44) pour absorber au moins brièvement le liquide,
**caractérisé en ce que**
le support (40) présente au moins par endroits la forme d'un cylindre creux (43) et l'élément de frottis (50) présente une tige (51), laquelle est insérée ou insérable au moins par endroits dans le cylindre creux (43), de sorte qu'une zone d'absorption (52) de l'élément de frottis (50) reliée à la tige (51) pour absorber le liquide dépasse du cylindre creux (43),
et
**que** l'élément de frottis (50) présente un premier tampon d'ouate (53) servant de zone d'absorption (52) sur la tige (51), dont l'étendue transversale maximale est supérieure au diamètre intérieur du cylindre creux (43).

2. Dispositif d'indication selon la revendication 1,
**caractérisé en ce que**
l'élément d'indication (10) est une bandelette indicatrice de pH pour la détermination optique du degré d'acidité du liquide.

3. Dispositif d'indication selon l'une des revendications 1 ou 2,
**caractérisé en ce que**
le support (40) est fabriqué à partir du même matériau que l'élément d'indication (10).

4. Dispositif d'indication selon au moins l'une des revendications 1 à 3,
**caractérisé en ce que**
l'élément de frottis (50) est fabriqué à partir d'un autre matériau que le support (40).

5. Dispositif d'indication selon au moins l'une des revendications précédentes,
**caractérisé en ce que**
l'élément d'indication (10) est raccordé directement à l'élément de frottis (50).

6. Dispositif d'indication selon au moins l'une des revendications précédentes,
**caractérisé en ce que**
l'élément de frottis (50) présente un tampon d'ouate (53, 54) de part et d'autre sur la tige (51).

7. Dispositif d'indication selon au moins l'une des revendications précédentes,
**caractérisé en ce que**
l'élément d'indication (10) est également conçu avec une tige (51) et un second tampon d'ouate (54) y étant raccordé, dans lequel la tige (51) de l'élément d'indication (10) est également insérée ou insérable dans le cylindre creux (43).

8. Dispositif d'indication selon au moins l'une des revendications précédentes,
**caractérisé en ce que**
la seconde extrémité du support (40) est conçue comme une poignée utilisable manuellement.

9. Dispositif d'indication selon au moins l'une des revendications précédentes,
**caractérisé en ce que**
le cylindre creux (43) présente un rétrécissement de sa section transversale creuse au moins en un point dans au moins une direction s'étendant perpendiculairement à la direction longitudinale du cylindre creux (43).

10. Dispositif d'indication selon au moins l'une des revendications précédentes,
**caractérisé en ce que**
le cylindre creux (43) et l'élément de frottis (50) présentent sensiblement le même diamètre.

11. Dispositif d'indication selon au moins l'une des revendications précédentes,
**caractérisé en ce que** l'élément de frottis (50) et/ou l'élément d'indication (10) et/ou le support (40) sont fabriqués à base d'un matériau biodégradable.

12. Utilisation du dispositif d'indication selon au moins l'une des revendications 1 à 11 pour indiquer au moins une valeur chimique d'un liquide et pour prélever simultanément un liquide en vue d'exécuter un frottis.
